# FASCICULE DE BREVET EUROPEEN

(11) **EP 3 320 035 B1**
(45) Date de publication et mention de la délivrance du brevet: **18.08.2021**
(21) Numéro de dépôt: 16736168.2
(22) Date de dépôt: 07.07.2016
(51) Int. Cl.: A61B 5/24, A61B 5/00, C08K 3/04

(54) **COMPOSITION POLYMÉRIQUE ET ÉLECTRODE POUR UN DISPOSITIF DE MESURE NON-INVASIVE DE SIGNAUX ÉLECTRIQUES BIOLOGIQUES**
POLYMERISCHE ZUSAMMENTSETZUNG UND ELEKTRODE FÜR EIN APPARAT FÜR EINE NICHTINVASIVE MESSUNG VON BIOLOGISCHEN ELEKTRISCHEN SIGNALEN
POLYMERIC COMPOSITION AND ELECTRODE FOR NON-INVASIVE MEASUREMENT DEVICE FOR BIOLOGICAL ELECTRICAL SIGNALS

(30) Priorité: 07.07.2015 FR 1556449
(43) Date de publication de la demande: 16.05.2018
(73) Titulaire: DREEM, 75009 Paris (FR)
(72) Inventeur: SOULET DE BRUGIERE, Quentin, 33115 Pyla sur Mer (FR); MERCIER, Hugo, 75008 Paris (FR)
(74) Mandataire: Plasseraud IP
(86) Numéro de dépôt international: PCT/EP2016/066131
(87) Numéro de publication internationale: WO 2017/005855

(56) Documents cités:
- GB-A- 2 447 640
- US-A- 4 763 659
- US-B1- 8 608 984
- DATABASE WPI Week 201548 Thomson Scientific, London, GB; AN 2015-40042A XP002761367, & CN 104 559 109 A (DONGGUAN CITY AONENG ENG PLASTIC CO LTD) 29 avril 2015 (2015-04-29)
- DATABASE WPI Week 201531 Thomson Scientific, London, GB; AN 2015-238617 XP002761368, & CN 104 371 153 A (JINING LEADER NANO TECHNOLOGY CO LTD) 25 février 2015 (2015-02-25)
- DATABASE WPI Week 201503 Thomson Scientific, London, GB; AN 2015-01005T XP002761369, & CN 104 086 783 A (UNIV HEILONGJIANG) 8 octobre 2014 (2014-10-08)

## Description

La présente invention est relative aux électrodes pour des dispositifs de mesure non-invasive de signaux électriques biologiques.

Plus particulièrement, l'invention se rapporte à une électrode pour un dispositif de mesure non-invasive de signaux électriques biologiques qui peut être utilisée sans gel conducteur et une composition polymérique pouvant notamment être utilisée dans une telle électrode.

Une électrode de mesure de signaux électriques biologiques est utilisée pour détecter et mesurer des bio-signaux par exemple représentatifs d'une activité nerveuse comme une activité cérébrale ou une activité musculaire.

De tels signaux électriques biologiques sont par exemple utilisés pour réaliser un électroencéphalogramme (EEG), un électromyogramme (EMG), un électrooculogramme (EOG) ou un électrocardiogramme (ECG), cette liste étant nullement limitative et trouvent des applications non seulement dans le domaine médical mais également dans des utilisations plus grand public, de loisir, de suivi personnel ou d'exercices.

On connaît des électrodes métalliques "humides", par exemple des électrodes Ag / AgCl, qui présentent de nombreux inconvénients parmi lesquels l'exigence d'une préparation de la peau avant contact par enlèvement des poils et grattage de la couche cornée, une irritation de la peau chez les personnes ayant la peau sensible lors d'une utilisation prolongée ou encore la présence d'un gel entre la peau et l'électrode peut compatible avec une utilisation de loisir régulière.

On connait par ailleurs des électrodes sèches métalliques, par exemple du document US 4865039. Toutefois de telles électrodes ont une qualité de signal moyenne, nécessitent un amplificateur coûteux et sont inconfortables lors d'utilisation prolongées.

Enfin, on connait, par exemple du document US 8608984, des électrodes polymériques sèches comportant un polymère thermoplastique polyuréthane à base de polyéther dans lequel sont dispersés un polymère de styrène et une charge conductrice comprenant un mélange de nanotubes de carbone et de noir de carbone.

La présente invention a notamment pour but d'améliorer les propriétés chimiques d'interface et les caractéristiques mécaniques et électriques de tels électrodes. La présente invention a par ailleurs pour but de proposer une électrode ne nécessitant pas de préparation préalable de la peau telles que lavage ou la présence d'un gel pour la collecte de signaux. Ledit matériau de capteur peut être utilisé avec ou sans contact direct avec la peau. Par ailleurs, la présente invention a pour but de proposer une électrode pouvant être aisément adaptée à l'utilisation voulu en ajustant les caractéristiques mécaniques, électriques et chimiques.

Ainsi, on définit une composition polymérique comprenant une matrice polymère dans laquelle sont dispersés des nanotubes de carbone et des particules de charbon activé.

Selon une réalisation, la composition polymérique comprend de 0,5 à 10 pourcent en poids de nanotubes de carbone, de préférence de 2 à 6 pourcent en poids de nanotubes de carbone, basé sur le poids total de la composition polymérique.

Selon une réalisation, la composition polymérique comprend de 0,5 à 30 pourcent en poids de particules de charbon activé, de préférence de 2 à 15 pourcent en poids de particules de charbon activé, basé sur le poids total de la composition polymérique.

Selon une réalisation, les particules de charbon activé présentent une surface spécifique supérieure à 500 m²/g, de préférence supérieure à 700 m²/g, encore plus préférentiellement supérieure à 1000 m²/g.

Selon une réalisation, la composition polymérique présente une dureté comprise dans une gamme allant de 10 shore A à 80 Shore A.

L'invention a pour objet une électrode pour la mesure non-invasive de signaux électriques biologiques, l'électrode comprenant une composition polymérique telle que décrite ci-avant apte à venir en contact avec un tissu vivant.

Selon une réalisation, la composition polymérique forme une première couche s'étendant entre une première face, apte à venir en contact avec un tissu vivant, et une deuxième face, opposée à la première face selon une direction d'épaisseur, et l'électrode comporte en outre une deuxième couche d'un polymère conducteur disposée sur la deuxième face de la composition polymérique.

Selon une réalisation, une épaisseur de la composition polymérique, mesurée selon la direction d'épaisseur, est inférieure à une épaisseur du polymère conducteur, de préférence au moins deux fois inférieure à une épaisseur du polymère conducteur.

Selon une réalisation, le polymère conducteur présente une dureté supérieure à une dureté de la composition polymérique.

L'invention a encore pour objet un circuit électrique pour la mesure non-invasive de signaux électriques biologiques comprenant
une électrode telle que décrite ci-avant, comprenant au moins un conducteur électrique relié à la composition polymérique ou au polymère conducteur, et
une unité de traitement des signaux mesurés par l'électrode relié au conducteur électrique de l'électrode.

L'invention a également pour objet un dispositif de mesure des ondes cérébrales apte à être porté par une personne, le dispositif comportant un élément support, apte à entourer au moins partiellement une tête d'une personne de sorte à y être maintenu, sur lequel est montée au moins une électrode telle que décrite ci-avant, de sorte à ce que la composition polymérique soit apte à venir en contact avec la peau de ladite personne.

Selon une réalisation, le dispositif comprend un circuit électrique tel que décrit ci-avant dans lequel l'électrode et l'unité de traitement sont montées sur élément support.

L'invention a enfin pour objet une utilisation de particules de charbon activé pour accroître l'adsorption ionique d'une composition polymérique comprenant une matrice polymère dans laquelle sont dispersés des nanotubes de carbone, lesdites particules de charbon activé étant dispersées dans la matrice polymère.

D'autres caractéristiques et avantages de l'invention apparaîtront au cours de la description suivante de plusieurs de ses formes de réalisation, données à titre d'exemples non limitatifs, en regard des dessins joints.

Sur les dessins :
- la figure 1 est une vue schématique d'une électrode selon un premier mode de réalisation de l'invention,
- la figure 2 est une vue schématique d'une électrode selon un deuxième mode de réalisation de l'invention, et
- la figure 3 est une vue schématique d'un dispositif de mesure des ondes cérébrales apte à être porté par une personne selon un mode de réalisation de l'invention.

Sur les différentes figures, les mêmes références désignent des éléments identiques ou similaires.

La composition polymérique 1 définie comporte une phase de dispersion et une phase dispersée. La phase de dispersion comporte une matrice polymère 2 et assure la structure mécanique de la composition tandis que la phase dispersée comporte des nanotubes de carbone 3 et des particules de charbon activé 4 et assure la conduction électrique dans la composition polymérique et l'interface électrochimique avec le tissu vivant 9 en contact avec la composition polymérique 1 ou l'électrode 6.

La matrice polymère 2 peut être un polymère unique ou un mélange de polymères. La matrice polymère est usuellement composée d'un ou d'une combinaison de polymères, silicones et hydrogels. La matrice polymère est choisie de sorte à présenter au moins l'une et ou l'autre caractéristiques suivantes : souple (dureté inférieure à 65 shore A), hydrophile, perméable aux ions Cl-, K+ et/ou Na+, compatible avec la peau et non allergène, résiste aux bactéries sur une durée d'au moins 6 mois, lavable à l'eau et/ou à l'alcool, et dont les propriétés mécaniques sont peu modifiées par l'ajout de carbone. La matrice polymère est par exemple choisie parmi les polymères de faible température de transition vitreuse.

La matrice polymère est par exemple choisie dans la liste des polymères tels que: le polyéthylène glycol et en particulier le poly(oxyéthylène) (PEG, POE), le polypropylène glycol et en particulier le polypropylene oxide (PPG, PPO), un copolymère de polyéthylène glycol et de polypropylène glycol, en particulier un co-polymère à trois-blocs comme un poloxamère, un alcool polyvinylique (PVOH, PVA, PVAl), un silicone comme le Polydimethylsiloxane (PDMS), un dérivé de cellulose et en particulier de l'hydroxypropyl méthylcellulose, de l'hydroxypropyl cellulose ou de la méthylcellulose (HPMC, HPC), un hydrogel.

En outre la matrice polymère peut contenir des modificateurs polymères et autres additifs comme par exemple des surfactants.

La matrice polymère 2 forme ainsi un matériau souple et amorphe, par exemple une matrice polymère élastomérique.

La matrice polymère est avantageusement hydrophile.

La composition polymérique 1 comprend par ailleurs des nanotubes de carbones 3 et des particules de charbon activé 4 dispersés dans la matrice polymérique.

Les nanotubes de carbone 3 utilisés dans la préparation des compositions polymériques ont par exemple un diamètre qui se situe dans la plage de 5 à 20 nanomètres et une longueur qui se situe dans la gamme de 1 à 5 microns.

Les nanotubes de carbone utilisés dans la préparation des compositions polymérique ont par exemple un rapport d'aspect qui est dans la plage de 80 à 200 mais peut aller jusqu'à 1000 ou plus.

Les nanotubes de carbone utilisés dans la préparation des compositions polymérique peuvent être des nanotubes de carbone à parois multiples (MWNT) ou des nanotubes de carbone à parois unique (SWNT).

La composition polymérique comprend par ailleurs des particules de charbon activé 4.

Par « charbon activé », on entend un matériau ayant subi une préparation particulière visant à lui conférer un fort pouvoir adsorbant, en particulier du fait d'une très grande surface spécifique.

Dans le cas de la présente divulgation, les particules de charbon activé utilisées peuvent notamment être préparées de sorte à présenter une forte adsorption des ions Cl-, K+ et/ou Na+ au moins.

Les particules de charbon activé peuvent notamment être préparées par carbonisation (pyrolyse) et/ou par activation/oxydation (exposition à une atmosphère oxydante). Les particules de charbon activé peuvent également, ou avant carbonisation, être préparées par activation chimique en étant imprégnées d'acide, de base forte ou d'un sel.

Par ailleurs, les particules de charbon activé sont usuellement hydrophobes. Les particules de charbon activé peuvent alors être traitées pour diminuer leur hydrophobicité de sorte à pouvoir plus aisément les disperser dans la matrice polymère.

Pour cela, il est par exemple possible de les faire tremper dans une solution d'éthanol puis de laisser l'éthanol s'évaporer.

Les particules de charbon activé ne sont pas du noir de carbone.

Les particules de charbon activé d'une composition polymérique définie peuvent présenter une surface spécifique supérieure à 500 m²/g, de préférence supérieure à 700 m²/g, encore plus préférentiellement supérieure à 1000 m²/g.

Les particules de charbon activé utilisées présentent par exemple une taille moyenne inférieure à 1 millimètre. Les particules de charbon activé utilisées sont par exemple des charbons activés en poudre, « Powdered activated carbon » (R 1, PAC), ou encore des charbons activés en billes, « Bead activated carbon » (BAC).

Dans certains modes de réalisation, les particules de charbon activé utilisées peuvent par ailleurs présenter une taille moyenne inférieure à 200 microns par exemple.

Les particules de charbon activé peuvent présenter une teneur en carbone inférieure à 90 % en poids.

Les particules de charbon activé sont de préférence dispersées dans la matrice polymère de manière homogène.

Par « dispersé de manière homogène », on entend que les particules de charbon activé ne forment pas d'agrégats, notamment pas d'agrégats ayant une taille supérieure à 1 millimètre.

Les communications électriques dans les tissus vivants, et notamment le corps humain, sont principalement réalisées par des transferts de charge ioniques comme les ions Cl-, K+ et/ou Na+. Ces déplacement de charges génèrent des modifications de potentiel électrique ou magnétique qui peuvent être mesurées jusqu'à l'extérieur du corps et donner une information sur son fonctionnement.

Pour mettre en œuvre une électrode, il est donc possible de capter les ions présents en surface du tissu vivant 9, notamment de la peau d'une personne 9, et de convertir ces ions en un déplacement électrique dans un circuit électrique de traitement de signal.

Pour cela, les particules de charbon activé fournissent une grande surface spécifique d'adsorption apte à adsorber une grande quantité des ions présents à la potentiel qui peut alors directement être transmise à un circuit électrique simplement connecté au potentiel de la surface du tissu vivant qui est en contact avec la composition polymérique.

Par ailleurs, les nanotubes de carbone permettent de rendre la composition polymérique conductrice.

L'adsorption des ions sur les particules de charbon activé provoque ainsi une modification du potentiel dans l'ensemble de la composition polymérique, modification de circuit électrique simplement connecté au potentiel de la composition polymérique.

A cet égard, les nanotubes de carbone permettent de rendre la composition polymérique conductrice avec un pourcentage de matière assez faible.

Ainsi notamment, le pourcentage en poids de nanotubes de carbone dans la matrice polymère correspondant à un seuil de percolation de conduction électrique des nanotubes de carbone dans la matrice polymère est en particulier inférieur à un pourcentage en poids d'éléments adsorbants dans la matrice polymère correspondant à un seuil de percolation de conduction électrique des éléments adsorbants dans la matrice polymère.

Pour fixer les idées en donnant un exemple purement non limitatif, le seuil de percolation de conduction électrique est usuellement atteint autour de 5% en poids de nanotubes de carbone dans une matrice polymère alors qu'il faut compter plus de 30% de poids de charbon actif pour former un réseau percolant.

L'intérêt d'incorporer une faible quantité de nanotubes de carbone et d'éléments adsorbants est de conserver les propriétés mécaniques de la matrice polymère.

Ainsi, avec une grande quantité d'éléments adsorbants, la composition polymérique matériau devient très rigide, ce qui diminue le confort et l'efficacité de l'électrode.

En effet, pour maximiser l'adsorption des ions dans la composition polymérique et le confort de l'utilisation, il est intéressant que la composition polymérique présente une dureté faible, de sorte à pouvoir aisément suivre les courbes d'un tissu vivant en contact, notamment une surface de peau.

Ainsi par exemple la composition polymérique comprend de 0,5 à 10 pourcent en poids de nanotubes de carbones, de préférence de 0,5 à 6 pourcent en poids de nanotubes de carbone, basé sur le poids total de la composition polymérique.

Par ailleurs, la composition polymérique peut comprendre de 0,5 à 30 pourcent en poids d'éléments adsorbants, de préférence de 0,5 à 15 pourcent en poids d'éléments adsorbants, basé sur le poids total de la composition polymérique.

De manière générale, un pourcentage en poids d'éléments adsorbants dans la composition polymérique peut être inférieur à un pourcentage en poids d'éléments adsorbants dans la matrice polymère correspondant à un seuil de percolation de conduction électrique des éléments adsorbants dans la matrice polymère.

La composition polymérique peut alors présenter une dureté comprise dans une gamme allant de 10 shore A à 80 Shore A.

La dureté de la composition polymérique peut notamment être inférieure à 65 shore A.

### Préparation :

La composition polymérique définie ci-dessus peut être préparée en utilisant une extrudeuse à vis double tournant dans le même sens, par exemple une extrudeuse à double vis de 25 mm de diamètre de la marque Berstorff, GmbH.

Les éléments de la composition polymérique peuvent être alimentés de plusieurs manières. Soit l'ensemble des éléments est fourni dans la gorge de l'extrudeuse et passe à travers ladite extrudeuse, soit les nanotubes de carbone et/ou les éléments adsorbants sont amenés par un dispositif d'alimentation latérale, et les éléments de la matrice polymérique sont amenés par la gorge. La composition polymérique peut ensuite être dégazée sous vide.

D'autres techniques de mélange à l'état fondu et de composition telles que des extrudeuses à vis unique et des mélangeurs de Banbury peuvent également être utilisés.

Dans un autre mode de réalisation de l'invention, la matrice polymérique peut être une matrice polymérique réticulable et les nanotubes de carbone et/ou les éléments adsorbants peuvent être mélangées à la matrice réticulable en phase liquide avant que la matrice ne soit réticulée en phase amorphe.

Dans certains mode de préparation, les éléments adsorbants et/ ou les nanotubes de carbone peuvent être fonctionnalisés avec des agents de couplage et/ou des agents de coiffage pour permettre leur dispersion homogène dans la matrice polymère.

La réticulation de la matrice polymère peut alors être réalisée par UV et/ou par chauffage. Un catalyseur peut être ajouté pour déclencher ou favoriser la réticulation, par exemple un photoamorceur radicalaire.

### Méthodes de mesure :

La surface spécifique des particules de charbon activé peut notamment être mesurée par la méthode de soustraction de l'effet des pores (« subtracting pore effect ») décrite dans l'article "Origin of superhigh surface area and microcrystalline graphitic structures of activated carbons." de Kaneko, K., C. Ishii, et M. Ruike, publié dans Carbon, vol 30.7 (1992): pages 1075-1088.

La surface spécifique des nanoplaquettes de graphène peut notamment être mesurée par la méthode Brunauer, Emmett et Teller (BET) d'adsorption d'azote, par exemple comme indiqué dans l'article « Processing of nanographene platelets (NGPs) and NGP nanocomposites: a review » de B. Z. Jang et A. Zhamu, publié dans Journal of Materials Science, août 2008, Volume 43.15, pages 5092-5101.

La dureté de la composition polymérique peut être mesurée à l'aide d'un duromètre Shore.

### Electrode :

L'invention a également pour objet une électrode 6 pour la mesure non-invasive de signaux électriques biologiques, notamment sans gel conducteur. L'électrode 6 comprend une composition polymérique 1 telle que décrite ci-avant, disposée de sorte à être apte à venir former une interface avec un tissu vivant 9.

Ainsi, la composition polymérique 1 peut être mise en forme dans l'électrode 6 pour former une première couche 1 s'étendant entre une première face la, apte à être en contact avec un tissu vivant 9, et une deuxième face 1b, opposée à la première face selon une direction d'épaisseur Z.

Dans un mode de réalisation de l'invention, la composition polymérique 1 peut être directement en contact avec des conducteurs électriques 5, notamment des fils électriques reliés à une unité de traitement 8 des signaux mesurés par l'électrode. Les conducteurs électriques 5 peuvent être incorporés dans la composition polymérique ou être placés en contact avec une face de la composition polymérique, par exemple la deuxième face 1b.

L'électrode 6 et l'unité de traitement 8 des signaux mesurés par l'électrode peuvent ainsi former un circuit électrique 7 pour la mesure non-invasive de signaux électriques biologiques.

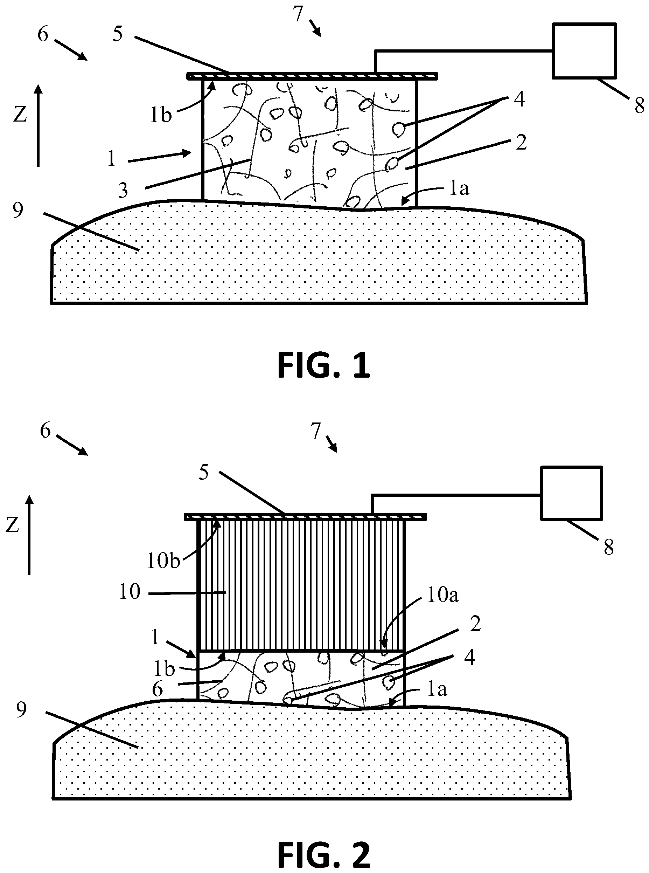

Dans une variante de réalisation illustrée sur la figure 2, l'électrode 6 peut comporter en outre une deuxième couche 10 d'un polymère conducteur 10. La deuxième couche peut être en contact avec la composition polymérique 1, en étant par exemple disposée sur la deuxième face 1b de la composition polymérique 1.

Dans cette variante de réalisation, la composition polymérique 1 peut être en contact avec les conducteurs électriques 5 par l'intermédiaire de la deuxième couche 10 de polymère conducteur.

Ainsi, les conducteurs électriques 5 peuvent être incorporés dans la deuxième couche 10 ou être placés en contact avec une face 10b de la deuxième couche 10.

En particulier, la deuxième couche 10 peut être mise en forme dans l'électrode 6 pour s'étendre entre une première face 10a en contact avec la deuxième face 1b de la première couche 1, et une deuxième face 10b, opposée à la première face 10a selon la direction d'épaisseur Z.

Les conducteurs électriques 5 peuvent alors être par exemple placés en contact avec la deuxième face 10b de la deuxième couche 10.

Dans un mode de réalisation, une épaisseur de la composition polymérique 1, mesurée selon la direction d'épaisseur Z, peut alors être inférieure à une épaisseur du polymère conducteur 10, mesurée selon la direction d'épaisseur Z. L'épaisseur de la composition polymérique 1 peut en particulier être au moins deux fois inférieure à l'épaisseur du polymère conducteur 10.

La présence d'une deuxième couche de polymère conducteur permet une plus grande liberté de propriétés mécaniques pour l'électrode.

Ainsi par exemple le polymère conducteur peut présenter une dureté supérieure à une dureté de la composition polymérique.

Dans un exemple de réalisation de l'invention, le polymère conducteur peut être un polyuréthane thermoplastique (TPU). Le polymère conducteur peut en particulier être dopé aux nanotubes de carbone de manière similaire à ce qui a été décrit ci-avant pour la composition polymérique, mais sans nécessiter la présence d'éléments adsorbants.

La composition polymérique et/ou le polymère conducteur peuvent être mis en forme par moulage par injection, par filage de fibres, par extrusion, ou encore par moulage par compression et par des combinaisons de ces techniques.

Dans le cas d'électrodes moulées par injection, une technique de surmoulage peut être utilisée dans laquelle des conducteurs électriques sont pré-placé dans un moule dans lequel est injecté la composition polymérique et/ou le polymère conducteur. Alternativement, une électrode peut être moulée, puis les conducteurs électriques sont soudés sur l'électrode, notamment sur la composition polymérique et/ou le polymère conducteur. De manière similaire, l'un parmi la composition polymérique et le polymère conducteur peut être moulé puis l'autre parmi la composition polymérique et le polymère conducteur peut être surmoulé.

Pour les électrodes extrudées, la composition polymérique et/ou le polymère conducteur peuvent être coextrudé ensemble et/ou avec les conducteurs électriques, puis en découpés en formes d'électrodes. Alternativement, l'un et/ou l'autre parmi la composition polymérique et/ou le polymère conducteur peut être extrudé sous forme de film, de feuille, de bande ou de mousse puis les conducteurs électriques peuvent être soudés ou introduits dans la composition polymérique et/ou le polymère conducteur. Les conducteurs électriques peuvent également être produits par des techniques de dépôt métallique sur la composition polymérique et/ou le polymère conducteur ou sur l'électrode formée.

Alternativement, la composition polymérique et/ou le polymère conducteur peuvent être formés en fibres et tissé dans un tissu ou des vêtements.

### Circuit électrique :

L'invention a encore pour objet un circuit électrique 7 incorporant une électrode 6 telle que décrite ci-avant comprenant au moins un conducteur électrique 5 relié électriquement à la composition polymérique 1 ou au polymère conducteur 10.

Le circuit électrique 7 comprend par ailleurs une unité de traitement 8 des signaux mesurés par l'électrode. L'unité de traitement 8 est reliée électriquement au conducteur électrique 5 de l'électrode 6 tels que décrits ci-avant.

L'unité de traitement 8 peut notamment comporter un amplificateur d'instrumentation et/ou des modules de filtration comme par exemple des filtres passe-bande ou passe-bas.

### Dispositif de mesure:

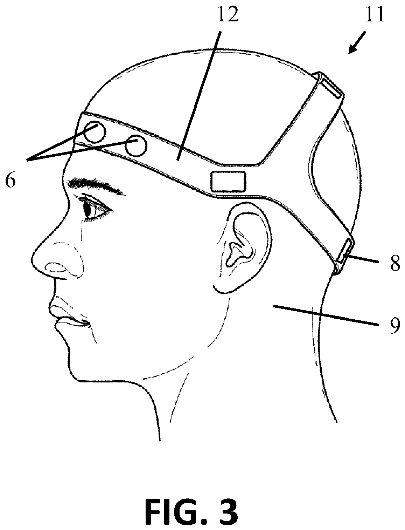

L'invention a enfin pour objet un dispositif de mesure 11 des ondes cérébrales apte à être porté par une personne 9, illustré schématique sur la figure 3. Le dispositif 1 comporte un élément support 12 apte à entourer au moins partiellement une tête d'une personne 9 de sorte à y être maintenu.

L'élément support 12 est apte à entourer au moins partiellement la tête de la personne 9 de sorte à y être maintenu. Dans un mode de réalisation de l'invention illustré sur la figure 1, l'élément support 12 est notamment apte à entourer au moins une portion d'une circonférence de la tête de la personne 9, notamment entourer au moins une moitié d'une circonférence de la tête de la personne 9, voire entourer en totalité un diamètre de la tête de la personne 9.

Dans le mode de réalisation illustré sur la figure 1, l'élément support 12 peut comporter plusieurs branches reliées entre elles qui entourent différentes portions de la tête de la personne 9 de sorte à assurer un maintien stable et une localisation précise du dispositif 11 sur la personne 9.

Au moins une électrode 6 telle que décrite ci-avant est montée sur l'élément support 12 de sorte à ce que la composition polymérique 1 soit apte à venir en contact avec la peau de la personne 9.

L'électrode 6 est par exemple montée sur une face intérieure de l'élément support 12 apte à venir en contact avec la peau de la personne.

Dans un mode de réalisation, le dispositif 11 comprend un circuit électrique 7 tel que décrit ci-avant. Dans ce mode de réalisation, l'unité de traitement 8 peut notamment être montée sur élément support 12.

## Revendications

1. Electrode (6) pour la mesure non-invasive de signaux électriques biologiques, l'électrode comprenant une composition polymérique (1) apte à venir en contact avec un tissu vivant, la composition polymérique (1) comprenant une matrice polymère (2) dans laquelle sont dispersés des nanotubes de carbone (3) et des éléments adsorbants (4) choisis parmi des particules de charbon activé et des nanoplaquettes de graphène.

2. Electrode selon la revendication 1, dans laquelle la composition polymérique forme une première couche (1) s'étendant entre une première face (1a), apte à venir en contact avec un tissu vivant, et une deuxième face (1b), opposée à la première face selon une direction d'épaisseur (Z), et dans laquelle l'électrode comporte en outre une deuxième couche (10) d'un polymère conducteur disposée sur la deuxième face (1b) de la composition polymérique.

3. Electrode selon la revendication 2, dans laquelle une épaisseur de la première couche (1), mesurée selon la direction d'épaisseur (Z), est inférieure à une épaisseur de la deuxième couche (10), de préférence au moins deux fois inférieure à une épaisseur de la deuxième couche (10).

4. Electrode selon l'une quelconque des revendications 2 et 3, dans laquelle le polymère conducteur présente une dureté supérieure à une dureté de la composition polymérique.

5. Circuit électrique (7) pour la mesure non-invasive de signaux électriques biologiques comprenant
- une électrode (6) selon l'une des revendications 1 à 4 comprenant au moins un conducteur électrique (5) en contact avec la composition polymérique ou le polymère conducteur,
- une unité de traitement (8) des signaux mesurés par l'électrode (6) reliée au conducteur électrique (5) de l'électrode (6).

6. Dispositif (11) de mesure des ondes cérébrales apte à être porté par une personne (9), le dispositif (11) comportant un élément support (12), apte à entourer au moins partiellement une tête de la personne de sorte à y être maintenu, sur lequel est montée au moins une électrode (6) selon l'une des revendications 1 à 4, de sorte à ce que la composition polymérique (1) de l'électrode (6) soit apte à venir en contact avec une peau de la personne.

7. Dispositif selon la revendication 6 comprenant un circuit électrique (7) selon la revendication 5 dans lequel l'électrode (6) et l'unité de traitement (8) sont montées sur élément support (12).

8. Utilisation d'éléments adsorbants choisis parmi des particules de charbon activé et des nanoplaquettes de graphène pour accroître l'adsorption ionique d'une composition polymérique comprenant une matrice polymère dans laquelle sont dispersés des nanotubes de carbone,
lesdits éléments adsorbants étant dispersés dans la matrice polymère.

## Patentansprüche

1. Elektrode (6) für die nichtinvasive Messung von biologischen elektrischen Signalen, wobei die Elektrode eine Polymer-Zusammensetzung (1) aufweist, die geeignet ist, um in Kontakt mit einem lebenden Gewebe zu kommen, wobei die Polymer-Zusammensetzung (1) eine Polymermatrix (2) aufweist, in der Kohlenstoffnanoröhrchen (3) und absorbierende Elemente (4), ausgewählt aus Aktivkohlepartikeln und Graphennanoplättchen, verteilt sind.

2. Elektrode nach Anspruch 1, in welcher die Polymer-Zusammensetzung eine erste Schicht (1) bildet, die sich zwischen einer ersten Fläche (1a), die mit einem lebenden Gewebe in Kontakt kommen kann, und einer der ersten Fläche in einer Dickenrichtung (Z) gegenüberliegenden zweiten Fläche (1b) erstreckt, und in welcher die Elektrode ferner eine zweite Schicht (10) eines leitfähigen Polymers aufweist, die an der zweiten Fläche (1b) der Polymer-Zusammensetzung angeordnet ist.

3. Elektrode nach Anspruch 2, in welcher eine Dicke der ersten Schicht (1), gemessen in der Dickenrichtung (Z), kleiner ist als eine Dicke der zweiten Schicht (10), vorzugsweise mindestens zwei Mal kleiner als eine Dicke der zweiten Schicht (10).

4. Elektrode nach einem der Ansprüche 2 und 3, in welcher das leitfähige Polymer eine Härte aufweist, die größer als eine Härte der Polymer-Zusammensetzung ist.

5. Elektrische Schaltung (7) für die nichtinvasive Messung von biologischen elektrischen Signalen, aufweisend:
- eine Elektrode (6) nach einem der Ansprüche 1 bis 4, die mindestens einen elektrischen Leiter (5) in Kontakt mit der Polymer-Zusammensetzung oder dem leitfähigen Polymer aufweist,
- eine mit dem elektrischen Leiter (5) der Elektrode (6) verbundene Verarbeitungseinheit (8) zum Verarbeiten der von der Elektrode (6) gemessenen Signale.

6. Vorrichtung (11) zum Messen von Gehirnwellen, die von einer Person (9) getragen werden kann, wobei die Vorrichtung (11) ein Trägerelement (12) aufweist, das einen Kopf der Person mindestens derart teilweise umgeben kann, dass es dort gehalten wird, und an dem mindestens eine Elektrode (6) nach einem der Ansprüche 1 bis 4 derart angebracht ist, dass die Polymer-Zusammensetzung (1) der Elektrode (6) in Kontakt mit einer Haut der Person kommen kann.

7. Vorrichtung nach Anspruch 6, aufweisend eine elektrische Schaltung (7) nach Anspruch 5, wobei die Elektrode (6) und die Verarbeitungseinheit (8) an dem Trägerelement (12) angebracht sind.

8. Verwendung von absorbierenden Elementen, ausgewählt aus Aktivkohlepartikeln und Graphennanoplättchen, zum Verstärken der Ionen-Adsorption einer Polymer-Zusammensetzung, die eine Polymermatrix aufweist, in der Kohlenstoffnanoröhrchen verteilt sind,
wobei die adsorbierenden Elemente in der Polymermatrix verteilt sind.

## Claims

1. An electrode (6) for non-invasive measurement of biological electrical signals, the electrode comprising a polymeric composition (1) adapted to come into contact with a living tissue, the polymeric composition (1) comprising a polymer matrix (2) in which carbon nanotubes (3) and adsorbing elements (4) selected from activated charcoal particles and graphene nanoplatelets are dispersed.

2. The electrode according to claim 1, wherein the polymeric composition forms a first layer (1) extending between a first face (1a), adapted to come into contact with a living tissue, and a second face (1b), opposite to the first face according to a thickness direction (Z), and wherein the electrode further includes a second layer (10) of a conductive polymer disposed over the second face (1b) of the polymeric composition.

3. The electrode according to claim 2, wherein a thickness of the first layer (1), measured according to the thickness direction (Z), is smaller than a thickness of the second layer (10), preferably at least two times smaller than a thickness of the second layer (10).

4. The electrode according to any one of claims 2 and 3, wherein the conductive polymer has a hardness higher than a hardness of the polymeric composition.

5. An electrical circuit (7) for non-invasive measurement of biological electrical signals comprising
- an electrode (6) according to one of claims 1 to 4 comprising at least one electrical conductor (5) in contact with the polymeric composition or the conductive polymer,
- a unit (8) for processing the signals measured by the electrode (6) connected to the electrical conductor (5) of the electrode (6).

6. A device (11) for measuring brainwaves adapted to be worn by a person (9), the device (11) including a support element (12), adapted to surround at least partially a head of the person so as to be held thereon, on which at least one electrode (6) according to one of claims 1 to 4 is mounted, so that the polymeric composition (1) of the electrode (6) is adapted to come into contact with a skin of the person.

7. The device according to claim 6 comprising an electrical circuit (7) according to claim 5 wherein the electrode (6) and the processing unit (8) are mounted on the support element (12).

8. A use of adsorbing elements selected from activated charcoal particles and graphene nanoplatelets to increase the ion adsorption of a polymeric composition comprising a polymer matrix in which carbon nanotubes are dispersed,
said adsorbing elements being dispersed in the polymer matrix.
